# EUROPEAN PATENT APPLICATION

(11) **EP 1 170 023 A2**
(43) Date of publication of application: **09.01.2002**
(21) Application number: 01116102.3
(22) Date of filing: 03.07.2001
(51) Int. Cl.: A61M 1/16

(54) **Hemodialysis machine**

(30) Priority: 04.07.2000 IT BO000395
(71) Applicant: BELLCO S.p.A., 41037 Mirandola (IT)
(72) Inventor: Cianciavicchia, Domenico, 64040 Cavuccio (IT)
(74) Representative: Bongiovanni, Simone (IT)

(57) **Abstract**

A hemodialysis machine (1) having at least one semipermanent component (12, 36) which is changed periodically after being used for several consecutive dialysis treatments; and a control unit (17) for controlling certain components (3, 10, 15) of the hemodialysis machine (1); the control unit (17) also monitoring operation of the components (3, 10, 15) to indicate any malfunctions endangering the patient; and the hemodialysis machine (1) being characterized by having means (18) for identifying semipermanent components (12, 36), and which provide for unequivocally identifying each semipermanent component (12, 36) on the machine (1), and for communicating its presence and identity to the control unit (17).

## Description

The present invention relates to a hemodialysis machine.

As is known, on so-called "hemodialysis machines" for the treatment of chronic renal failure, the patient's blood is fed through a blood purifying unit by which most of the impurities in the blood are extracted by osmosis. On some types of hemodialysis machines, the treated blood, before being transfused, is also mixed with a reinfusion liquid to restore the solutes dissolved in it and re-establish a suitable water balance, which is seriously impaired in the passage through the blood purifying unit.

The blood purifying unit of such machines normally comprises a capillary-fiber filter in which the patient's blood is brought into contact with a dialysis liquid, via the interposition of a semipermeable membrane, so that the toxic substances in the blood pass from the patient's blood to the dialysis liquid by osmosis; a dialysis liquid reservoir; pumping means for feeding the dialysis liquid from the reservoir to the capillary-fiber filter and from the capillary-fiber filter to a drain conduit; and, finally, a first filtering element for filtering the dialysis liquid upstream from the capillary-fiber filter.

Hemodialysis machines also comprise pumping means for withdrawing the patient's blood, feeding it through the capillary-fiber filter, and, finally, feeding it back into the patient's bloodstream; and a drip device, downstream from the capillary-fiber filter, for removing any air from the purified blood which may result in an embolism or other serious pathologies.

When designed to restore the solutes dissolved in the blood and re-establish a suitable water balance, currently marketed hemodialysis machines also comprise a reinfusion liquid reservoir; and pumping means for feeding the reinfusion liquid from the reservoir to the drip device where the reinfusion liquid is normally mixed with the patient's blood.

Given the similarity of the reinfusion and dialysis liquids, most hemodialysis machines use the dialysis liquid itself as a reinfusion liquid, and therefore have no reinfusion liquid reservoir, and the reinfusion liquid pumping means are connected to the hydraulic circuit feeding the dialysis liquid to the capillary-fiber filter, so as to also feed the dialysis liquid to the drip device. In this case, the hemodialysis machine also comprises a second filtering element for filtering the reinfusion liquid upstream from the drip device, to prevent the patient from coming into contact with any toxic substances suspended in the reinfusion liquid.

Hemodialysis machines also comprise a unit for preparing the dialysis liquid, and which comprises a source of saline concentrates for supply to the reservoir; a source of water for supply to the reservoir; and a third filtering element for filtering the water upstream from the reservoir where it is mixed with the saline concentrates to form the dialysis liquid.

To ensure maximum safety of the patient, the capillary-fiber filter, the tubes connecting the capillary-fiber filter to the dialysis liquid reservoir, the tubes connecting the capillary-fiber filter to the patient, the drip device, the reinfusion liquid filtering element, and the tubes connecting the filtering element to the drip device are all disposable to prevent any toxic substances or bacteria in the treated blood from being transferred to the untreated blood of the patient.

On the other hand, for cost reasons, the first and third filtering elements and possibly other parts of the machine are "semipermanent", i.e. are changed at regular intervals, after being used for more than one dialysis treatment. Needless to say, at the end of each dialysis treatment, the first and third filtering elements are sterilized thoroughly.

Unfortunately, hemodialysis machines of the type described above have the major drawback of failing to provide for determining due replacement of the first and/or third filtering element, thus exposing the patient to the risk of being treated using deteriorated, i.e. unreliable, machine parts.

It is an object of the present invention to provide a hemodialysis machine designed to eliminate the aforementioned drawbacks.

According to the present invention, there is provided a hemodialysis machine comprising at least one semipermanent component which is changed periodically after being used for several consecutive dialysis treatments; and a control unit for controlling certain components of said hemodialysis machine; said control unit also monitoring operation of said components to indicate any malfunctions endangering the patient; and said hemodialysis machine being characterized by comprising means for identifying semipermanent components, and which provide for unequivocally identifying the semipermanent component on the machine, and for communicating its presence and identity to said control unit.

A non-limiting embodiment of the present invention will be described by way of example with reference to the accompanying drawings, in which:
Figure 1 shows a schematic, with parts removed for clarity, of a hemodialysis machine in accordance with the teachings of the present invention;
Figure 2 shows a detail of the Figure 1 hemodialysis machine;
Figures 3 and 4 show schematics of two Figure 2 components of the hemodialysis machine.

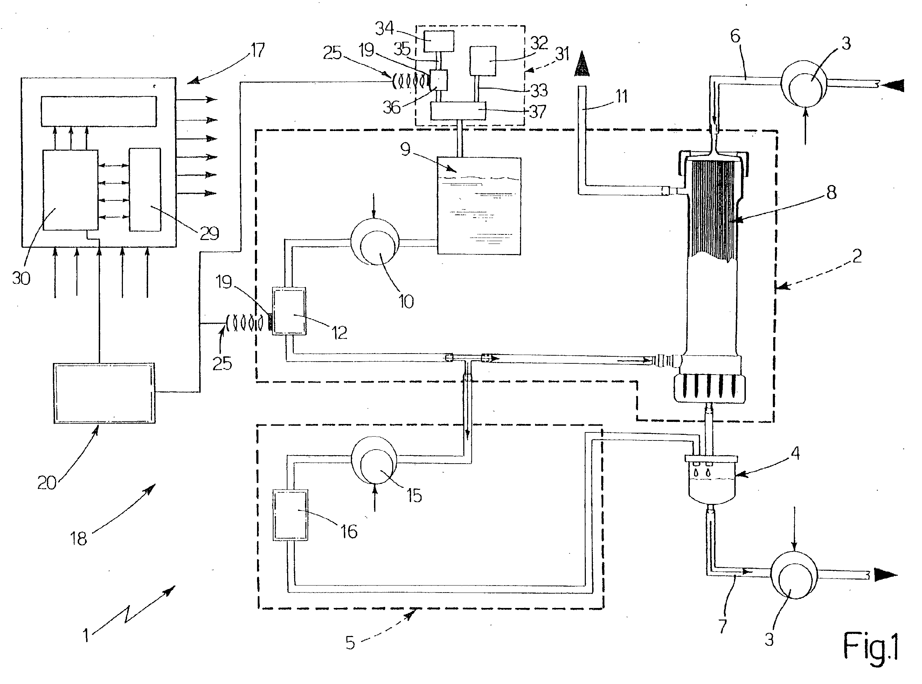

Number 1 in Figure 1 indicates as a whole a so-called hemodialysis machine for treating patients suffering from chronic renal failure.

Hemodialysis machine 1 comprises a blood purifying unit 2, through which the patient's blood is fed to remove any toxic solutes; a blood supply circuit connecting purifying unit 2 to the patient; one or more circulating pumps 3 for maintaining a given blood flow rate through purifying unit 2; and a known drip device 4 for eliminating from the blood issuing from purifying unit 2 any air which, on reaching the patient, could result in an embolism or other serious pathologies.

Preferably, though not necessarily, hemodialysis machine 1 also comprises a liquid restoring device 5 for mixing with the blood issuing from purifying unit 2 a saline solution, commonly referred to as a "reinfusion liquid", to re-establish the right proportion of salt solution in the blood and also restore part of the liquid withdrawn from the blood by purifying unit 2.

The blood supply circuit comprises two conduits 6 and 7 made of plastic material : the first for feeding the blood from an artery of the patient to purifying unit 2; and the second for feeding the blood purified in unit 2 into a vein of the patient.

In the example shown, a circulating pump 3, preferably though not necessarily a peristaltic type, is installed along each conduit 6 and 7.

With reference to Figure 1, purifying unit 2 comprises a known capillary-fiber filter 8 in which the patient's blood is brought into contact with a dialysis liquid, via the interposition of a semipermeable membrane, so that the toxic substances in the blood pass by osmosis from the patient's blood to the dialysis liquid; a dialysis liquid reservoir 9; a circulating pump 10, preferably though not necessarily a peristaltic type, for feeding the dialysis liquid from reservoir 9 to capillary-fiber filter 8; a drain conduit 11 along which the contaminated dialysis liquid containing the toxic solutes withdrawn from the blood is drained from capillary-fiber filter 8; and a filtering element 12 for filtering the dialysis liquid from reservoir 9 to retain any suspended impurities upstream from capillary-fiber filter 8.

Hemodialysis machine 1 preferably, though not necessarily, also comprises a unit 31 for preparing the dialysis liquid. Unit 31 comprises a saline concentrate source 32 connected by a conduit 33 to a mixing device 37 in turn connected to reservoir 9; a water source 34 connected by a conduit 35 to mixing device 37; and a filtering element 36 installed along conduit 35 to filter the water fed along conduit 35. The saline concentrates and water are normally fed by means of appropriate pumps.

In the example shown, capillary-fiber filter 8 is defined by an airtight container housing a bundle of capillary tubes made of semipermeable material. The container is connected to drain conduit 11 and to circulating pump 10 so as to be kept full of dialysis liquid at all times; and the bundle of capillary tubes connects conduits 6 and 7 so that, as the patient's blood flows along the capillary tubes, the toxic substances in suspension are transferred by osmosis to the dialysis liquid flowing over the outer surface of the capillary tubes.

With reference to Figure 1, liquid restoring device 5 comprises a reinfusion liquid reservoir, which, in the example shown, coincides with dialysis liquid reservoir 9; a circulating pump 15, preferably though not necessarily a peristaltic type, for feeding part of the dialysis liquid issuing from filtering element 12 to drip device 4; and a filtering element 16 for filtering the dialysis liquid issuing from circulating pump 15.

Blood supply circuit conduits 6 and 7, drip device 4, capillary-fiber filter 8, filtering element 16, and the conduit for feeding the reinfusion liquid to drip device 4 are preferably, though not necessarily, disposable, i.e. are changed after each dialysis treatment; whereas filtering elements 12 and 36, and possibly other component parts, not shown, of machine 1, are "semipermanent", i.e. are changed at regular intervals, after being used for various consecutive dialysis treatments.

Hereinafter, filtering element 12, filtering element 36, if present, and any other "semipermanent" component parts of machine 1 will be referred to simply as semipermanent components.

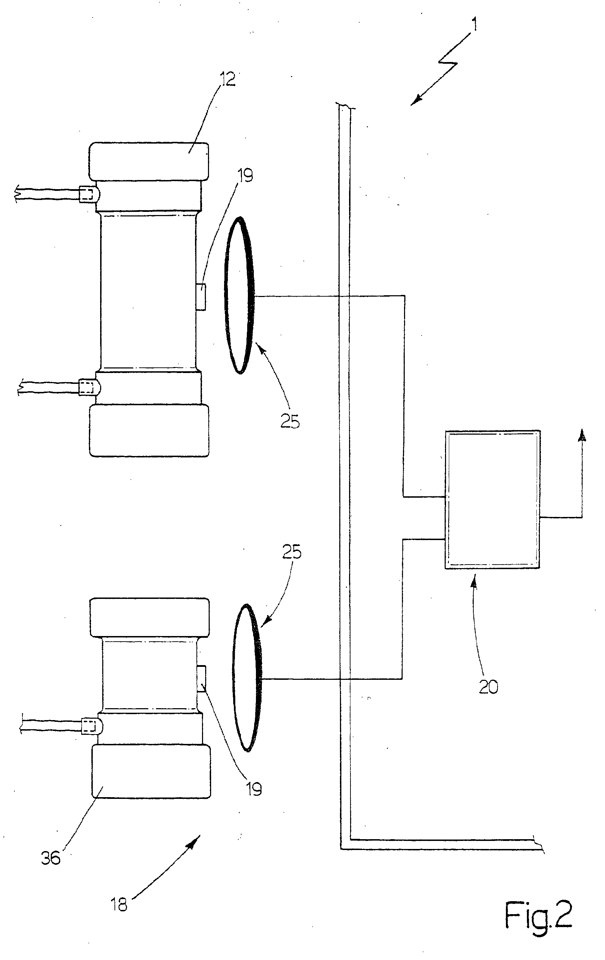

With reference to Figures 1 and 2, hemodialysis machine 1 also comprises an electronic central control unit 17 for controlling circulating pumps 3, 10 and 15 and generally monitoring operation of the various component parts of machine 1 to indicate any malfunctions endangering the patient; and a semipermanent-component identification device 18 for unequivocally identifying the various semipermanent components on machine 1, and for communicating their presence and identity to electronic central control unit 17.

In the example shown, each semipermanent component fitted or fittable to machine 1, i.e. each existing filtering element 12 or 36, is assigned an "identification code" unequivocally identifying the component; and identification device 18 is designed to detect and communicate the identification code to electronic central control unit 17 to enable the control unit to determine whether or not any one of the semipermanent components has been changed. In the event the component has not been changed, electronic central control unit 17 obviously provides for disabling operation of machine 1.

With reference to Figures 1, 2 and 3, in the example shown, semipermanent-component identification device 18 comprises a known transponder 19 fitted irremovably to the body of each semipermanent component fitted or fittable to machine 1, i.e. to each existing filtering element 12 or 36, and in which the component identification code is memorized; and a transponder reading unit 20, which dialogs with the transponder 19 of each semipermanent component on machine 1 to determine the identification code of the component.

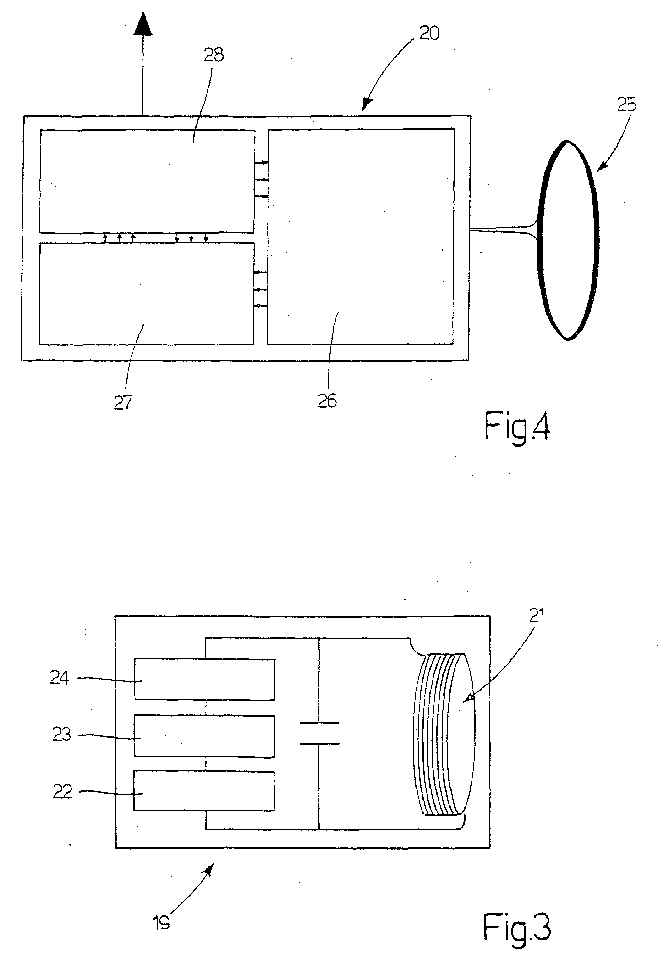

With reference to Figure 3, transponder 19 in the example shown comprises: a miniaturized transmitter-receiver antenna 21; a miniaturized transmitter-receiver 22 for exchanging radiofrequency signals with transponder reading unit 20; a permanent memory 23 storing the identification code of the semipermanent component to which transponder 19 is fitted; and a control unit 24 for driving transmitter-receiver 22 to transmit, when requested, the identification code in permanent memory 23.

With reference to Figure 4, reading unit 20 in the example shown comprises one or more transmitter-receiver antennas 25 located close to the semipermanent component/s on machine 1, i.e. close to filtering element 12 and filtering element 36, if present; a transmitter-receiver 26 for exchanging radiofrequency signals with the transponders 19 of the semipermanent components on machine 1 via antennas 25; a decoder 27 for determining, on the basis of the incoming radiofrequency signals, the identification codes of the semipermanent components on machine 1, i.e. the identification codes of filtering element 12 and filtering element 36, if present; and a control unit 28 for driving transmitter-receiver 26 and decoder 27, when commanded by electronic central control unit 17, to determine the identification code of each transponder 19 and communicate the identification code to control unit 17.

With reference to Figure 1, electronic central control unit 17 comprises a permanent memory 29 storing the identification codes of the semipermanent components which have been or are currently fitted to machine 1; and a control unit 30 for informing the operator when to change any one semipermanent component, and for determining that the identification code of the newly fitted semipermanent component has not been fitted to machine 1 before. If the identification code of the new semipermanent component is not already stored in permanent memory 29, control unit 30 updates permanent memory 29; conversely, it disables machine 1.

Operation of hemodialysis machine 1 as described and illustrated herein will be clear from the foregoing description, with no further explanation required.

The advantages of hemodialysis machine 1 as described and illustrated herein are obvious: when electronic central control unit 17 commands replacement of any. one semipermanent component, the machine can no longer be started if the same semipermanent component is removed and reassembled.

Clearly, changes may be made to hemodialysis machine 1 as described and illustrated herein without, however, departing from the scope of the present invention.

## Claims

1. A hemodialysis machine (1) comprising at least one semipermanent component (12, 36) which is changed periodically after being used for several consecutive dialysis treatments; and a control unit (17) for controlling certain components (3, 10, 15) of said hemodialysis machine (1); said control unit (17) also monitoring operation of said components (3, 10, 15) to indicate any malfunctions endangering the patient; and said hemodialysis machine (1) being **characterized by** comprising means (18) for identifying semipermanent components (12, 36), and which provide for unequivocally identifying the semipermanent component (12, 36) on the machine (1), and for communicating its presence and identity to said control unit (17).

2. A hemodialysis machine as claimed in Claim 1, **characterized in that** said control unit (17) indicates the need to change said semipermanent component (12, 36), and disables the hemodialysis machine (1) in the event the identity of the new semipermanent component (12, 36) matches the identity of any one of the semipermanent components (12, 36) previously fitted to the hemodialysis machine (1).

3. A hemodialysis machine as claimed in Claim 1 or 2, **characterized in that** said means (18) for identifying semipermanent components (12, 36) comprise a transponder (19) in which is memorized an identification code associated unequivocally with the semipermanent component (12, 36), and which is fitted irremovably to the body of the semipermanent component (12, 36); and a transponder reading unit (20) which dialogs with said transponder (19) to determine the identification code of the semipermanent component (12, 36).

4. A hemodialysis machine as claimed in any one of the foregoing Claims, **characterized by** comprising a purifying unit (2) through which the patient's blood is fed to remove any dissolved toxic elements; a blood supply circuit (6, 7) connecting said purifying unit (2) to the patient; and first pumping means (3) for circulating the patient's blood through the purifying unit (2); said control unit (17) driving said first pumping means (3) to regulate the blood flow through the purifying unit (2).

5. A hemodialysis machine as claimed in Claim 4, **characterized in that** said purifying unit (2) comprises a capillary-fiber filter (8) in which the patient's blood is brought into contact with a dialysis liquid via the interposition of a semipermeable membrane; a reservoir (9) for said dialysis liquid; second pumping means (10) for feeding the dialysis liquid from the reservoir (9) to the capillary-fiber filter (8); and a filtering element (12) for filtering the dialysis liquid issuing from said reservoir (9) to retain, upstream from said capillary-fiber filter (8), any impurities suspended in the dialysis liquid; said at least one semipermanent component (12, 36) comprising said filtering element (12).

6. A hemodialysis machine as claimed in Claim 5, **characterized by** comprising a unit (31) for preparing the dialysis liquid, and which comprises a saline-concentrate source (32) connected to said reservoir (9); a water source (34) connected to said reservoir (9); and a second filtering element (36) for filtering the water fed to said reservoir (9); said at least one semipermanent component (12, 36) comprising said second filtering element (36).
